# FASCICULE DE BREVET EUROPEEN

(11) **EP 1 110 941 B1**
(45) Date de publication et mention de la délivrance du brevet: **17.03.2004**
(21) Numéro de dépôt: 00403478.1
(22) Date de dépôt: 11.12.2000
(51) Int. Cl.: C07C 67/347, C07C 69/587

(54) **Procédé pour la codimérisation de corps gras polyinsaturés et d'oléfines par des complexes au fer**
Verfahren zur Codimerisierung von polyungesättigten Fettverbindungen und Olefinen mit Eisencomplexen
Process for the codimerisation of polyunsaturated fatty compounds and olefines with iron complexes

(30) Priorité: 24.12.1999 FR 9916508
(43) Date de publication de la demande: 27.06.2001
(73) Titulaire: Institut Français du Pétrole, 92852 Rueil-Malmaison Cedex (FR)
(72) Inventeur: Bach, Ingrid, 92500 Rueil Malmaison (FR); Hillion, Gérard, 95220 Herblay (FR); Olivier, Hélène, 92500 Rueil Malmaison (FR)

(56) Documents cités:
- FR-A- 2 766 482

## Description

L'invention a pour objet un nouveau procédé d'obtention de composés chimiques issus de corps gras polyinsaturés, composés caractérisés par la présence le long de la chaîne linéaire hydrocarbonée d'une ramification de deux atomes de carbone au moins.

Ces composés sont obtenus par addition d'oléfines sur des corps gras polyinsaturés conjugués ou non, en présence d'un système catalytique au fer.

Les co-dimères insaturés obtenus peuvent être hydrogénés et l'on obtient alors des corps gras saturés caractérisés par un point de fusion généralement inférieur à -30°C, une thermostabilité importante et des propriétés tensioactives recherchées.

La présence de ramifications dans les composés à base de corps gras, surtout lorsque ces ramifications se situent vers le milieu des chaînes linéaires comportant de 14 à 18 atomes de carbone, se traduit par un certain nombre de propriétés remarquables comme par exemple :
- L'abaissement très important des points de fusion, des points d'écoulement, des points de trouble et une augmentation sensible de la viscosité des corps gras ramifiés par rapport aux mêmes composés linéaires (non ramifiés). Cette propriété est par exemple utilisée dans les lubrifiants, les graisses, les plastifiants où l'on emploie des esters de corps gras, des sels ou des esters d'alcools ramifiés dont l'acide peut être organique ou minéral.
- La réduction de la tension superficielle et interfaciale, caractéristiques toujours recherchées dans le domaine des tensioactifs et des émulsifiants. Cette réduction permet d'obtenir des CMC (Concentration Micellaire Critique) très basses.
- L'inhibition de la cristallisation des savons ramifiés en mélange ou non avec des savons classiques, ce qui permet d'obtenir des savons transparents.
- Une augmentation de l'hydrophilie, ce qui rend les composés ramifiés plus solubles ou plus mouillables. Une utilisation possible serait d'utiliser des sels quaternaires d'acides gras ramifiés dans les adoucissants où l'adoucissement va de pair avec une certaine mouillabilité.
- Une modification de la surface de la molécule, surface caractérisée par des vides qui sont provoqués par la présence de ramifications. L'application en cosmétique de cette propriété permet d'envisager des formules de crèmes pour la peau qui laissent passer la vapeur d'eau, par exemple des bases constituées d'esters d'acides ramifiés ou même d'esters dans lesquels l'acide et l'alcool sont tous deux ramifiés.
- La solubilité accrue des sels de métaux lourds avec des acides ramifiés, ce qui les rend solubles soit dans l'eau soit dans certains solvants organiques. Les applications sont multiples comme agents siccatifs dans les peintures, comme pigments, dans l'extraction des métaux, l'anticorrosion où l'on peut utiliser des sels de calcium, d'alcanolamines ou même d'amines comme agents actifs. De même, les sels d'acides ramifiés offrent une compatibilité plus grande de certaines charges minérales avec des polymères, ce qui permet d'augmenter le taux des charges dans les plastiques.
- L'effet bactéricide ou bactériostat plus ou moins marqué selon la nature des bactéries et le nombre ou la grandeur des ramifications permet de protéger des crèmes contre l'attaque bactérienne ou de remplacer les sels quaternaires dans les formulations basiques ou non. Une autre utilisation existe comme inhibiteur d'évaporation de l'eau où par exemple des composés comme un alcool ramifié ou un monoglycéride d'acide ramifié, permettent de retarder la biodégradabilité donc d'économiser l'inhibiteur.

La réaction des oléfines avec le butadiène ou d'autres diènes est connue depuis longtemps et a fait l'objet de plusieurs revues. La codimérisation du butadiène avec l'éthylène conduit au 1,4-hexadiène, celle de l'éthylène avec l'isoprène au méthyl-3 hexadiène et enfin, par codimérisation de l'éthylène avec le pipérylène, on obtient le vinyl-2-pentène. De nombreux catalyseurs sont utilisés pour réaliser ces réactions. On peut citer par exemple les systèmes au rhodium, au ruthénium, au palladium, au cobalt, au fer ou au nickel. Les systèmes à base de titane ont été décrits (Connel, Laurence G.- Ann. N.Y. Acad. Sci.(73), 214, 143-9) pour catalyser la formation du vinylcyclobutane à partir d'éthylène et de butadiène.

Le brevet US-A-3 927 137 et la demande de brevet allemand DE-A-39 06 434 décrivent l'utilisation de systèmes catalytiques à base des sels de fer associés à des ligands de types imines ou diimines pour codimériser des α-oléfines de bas poids moléculaires avec des dioléfines conjuguées.

Par contre, l'addition d'une oléfine sur des diènes fonctionnels n'a été que très peu décrite. Le brevet US-A-3 742 080 signale la possibilité d'additionner de l'éthylène à des diènes dont l'une ou les deux extrémités de chaînes hydrocarbonées sont substituées par des atomes d'halogènes ou des groupes alkoxy.

Il est également connu qu'une oléfine peut réagir sur un composé diénique ou triénique conjugué selon une réaction de type Diels-Alder. Par exemple R.E. Beal et Coll. [JAOCS 52, 400 (1975)] ont décrit l'addition d'éthylène sur des corps gras polyinsaturés par simple chauffage à une température de 290 °C. Ainsi, à partir de linoléate de méthyle et d'éthylène, on obtient un composé possédant dans sa chaîne hydrocarbonée un cycle insaturé à 6 atomes de carbone. Après hydrogénation, ces composés possèdent des propriétés intéressantes. Toutefois, leur point de fusion, supérieur à 10 °C, est encore trop élevé pour permettre leur utilisation comme lubrifiants.

On connaît une autre méthode d'obtention de composés ramifiés de corps gras. Elle consiste à faire réagir, selon une réaction de type Wittig, une cétone comme par exemple l'ester méthylique de l'acide 12- oxo octadécanoïque avec un ylure, par exemple l'intermédiaire P(⌀)₃=CHCH₃, où ⌀ représente un radical phényle. On obtient alors le composé CH₃(CH₂)₅C(=CHCH₃)(CH₂)₁₀COOCH₃, qui peut être hydrogéné en éthyl-12-octadécanoate de méthyle. [voir D.G. Chasin et Coll., Chem. Phys. Lipids (71) 6, 8-30].

On a signalé également dans la nature la présence de composés saturés ramifiés de corps gras tirés des bacilles de Koch par exemple, ou, avec une autre longueur de chaîne hydrocarbonée, dans le suif de mouton.

On sait enfin que les produits que l'on appelle «isostéariques» contiennent des traces de composés portant des ramifications de type éthyle ou vinyle.

Récemment, les demandes de brevets internationales WO-A-91/11428, 91/11427, 91/11426 et 91/11425 décrivent l'obtention de composés de corps gras ramifiés par un procédé catalytique. L'addition de l'oléfine, telle que l'éthylène, le propylène ou le butène-1, sur le corps gras polyinsaturé, un ester de l'acide linoléique par exemple, est catalysée par un système à base de rhodium, d'iridium, de palladium ou de ruthénium. Les systèmes au rhodium, qui sont les seuls à avoir été décrits de façon évidente, restent cependant peu actifs.

Les brevets US-A-5 476 956 et 5 434 282 décrivent l'utilisation d'un système catalytique au rhodium très spécifique, qui permet d'accélérer l'addition de l'oléfine sur les diènes de corps gras, notamment les diènes conjugués, d'un facteur 50 à 100. Toutefois, ce procédé reste encore difficilement applicable à une grande échelle en raison de la consommation trop élevée de rhodium.

Le brevet FR-B-2 766 482, au nom de la demanderesse, décrit un système catalytique au cobalt qui consiste à faire réagir des oléfines simples, par exemple l'éthylène et le propylène, sur des esters polyinsaturés, par exemple le linoléate de méthyle conjugué ou non, pour obtenir des esters ramifiés. Les composés ramifiés obtenus peuvent être hydrogénés et être utilisés, entre autres, comme bases de lubrifiants. Dans cette demande, on décrit un procédé d'obtention d'un codimère. Des co-catalyseurs peuvent éventuellement être introduits, comme par exemple des métaux de transition du type fer, nickel, cuivre, rhodium ou palladium. Ces co-catalyseurs permettent de catalyser la conjugaison, si l'on part d'un ester polyènique non conjugué, et donc d'accélérer la vitesse de réaction.

L'amélioration principale qu'apporte la présente invention consiste en une augmentation très significative de réactivité obtenue par utilisation d'un système catalytique à base de fer par rapport au système précédent qui utilisait un système principalement au cobalt. En travaillant à isoconditions, on obtient 10 à 15 fois plus de produits de monoaddition.

D'un point de vue économique, le système catalytique décrit dans la présente invention pourrait devenir très intéressant pour déboucher sur un éventuel développement industriel.

Le composé dit «corps gras polyinsaturé» mis en jeu dans la réaction sur laquelle est basée le procédé de l'invention est en général un composé comprenant, d'une part, au moins deux liaisons éthyléniques, ces liaisons pouvant être conjuguées ou conjugables deux à deux et, d'autre part, une fonction carboxylique comme celle présente dans les acides gras ayant de 18 à 26 atomes de carbone. Les huiles de tournesol, de carthame, de poisson, de lin, de soja, d'oïticica, de coton, de colza, de bois de chine, de noix, de maïs, de linola, de pépins de raisin et généralement toutes les huiles ou leurs esters dérivés comprenant des composés polyinsaturés sont des matières premières envisageables.

Les acides gras considérés, diéniques, triéniques ou polyéniques peuvent être utilisés tels quels ou de préférence sous la forme de leurs esters, que l'on forme soit à partir d'acides gras ou d'huiles par réaction avec des alcools monofonctionnels, tels que le méthanol ou l'éthanol, difonctionnels, tels que le néopentylglycol, trifonctionnels, tels que le triméthylolpropane, polyfonctionnels, tels que le sorbitol, les polyglycérols, le pentaérythritol et les sucres. Les huiles elles-mêmes sont des substrats possibles.

Ces esters peuvent être utilisés tels quels ou conjugués partiellement et/ou totalement. Autrement dit, ils peuvent contenir au moins deux doubles liaisons éthyléniques séparées ou non entre elles par un groupe méthylénique. Parmi les procédés de conjugaison des doubles liaisons les plus connus, on peut citer ceux qui utilisent les alcoolates alcalins en présence ou non d'un solvant. On peut dans ce cas obtenir jusqu'à 99 % de corps gras conjugué par rapport au corps gras polyinsaturé présent initialement dans l'huile.

On connaît d'autres systèmes catalytiques conjugants, mettant en jeu des complexes de ruthénium ou le fer carbonyle. Le système au fer peut être dans certains cas lui-même conjugant. On peut cependant lui adjoindre un co-métal pour accélérer la réaction de conjugaison.

Le composé monooléfinique mis en jeu dans la réaction peut consister en toute oléfine réactive choisie parmi les monooléfines ordinaires (hydrocarbures monooléfiniques) telles que par exemple l'éthylène, le propylène ou le butène-1.

L'objet de la présente invention est donc un nouveau procédé d'obtention d'un corps gras ramifié, sous forme d'un codimère, caractérisé en ce que l'on additionne au moins un composé monooléfinique sur un corps gras comportant au moins deux liaisons éthyléniques conjuguées ou non conjuguées, en présence d'un système catalytique comprenant au moins un composé du fer et éventuellement, en proportion mineure, au moins un sel d'au autre métal de transition, au moins un composé réducteur et au moins un ligand contenant du phosphore, de l'arsenic, de l'antimoine ou de l'azote.

Le composé du fer peut être un composé inorganique ou organique bivalent ou trivalent de fer qui répond à la formule Fe X_{n,} dans laquelle n = 2 ou 3, et X représente un halogénure, un thiocyanure, un sulfate, un nitrate, un alcoolate, un carbonate, un carboxylate, une dicétone, un ester d'acide bétacétocarboxylique, un hydroxyle, un groupe alkyle ou alkényle (dans les composés organo-fer) ou encore un hydrure. Des exemples particuliers de composés de fer utilisables sont le bisacétylacétonate de fer (II), le trisacétylacétonate de fer (III) et l'octoate de fer (II) et (III) ;

Les composés réducteurs du fer utilisables sont choisis le plus souvent parmi :
- les composés organoaluminiques de formule générale AIRₓ(X)₃₋ₓ, où R est l'hydrogène ou un groupe alkyle par exemple méthyle, éthyle, isopropyle, butyle, isobutyle ou terbutyle, ou un groupement alcoxy ; X est un atome d'halogène ; et x est égal à 1 ou 2 ,
- les organo-magnésiens, les aluminoxanes, le borhydrure de sodium et les hydrures alcalins variés, tels que LiAlH₄ et NaAlH₄ eux-mêmes ou leurs dérivés obtenus en substituant de 1 à 3 atomes d'hydrogène par 1, 2 ou 3 groupements alcoxy, par exemple LiAlH₃(OR), LiAlH₂(OR)₂ et LiAlH(OR)₃, où R est un groupement alkyle, par exemple méthyle, éthyle, isopropyle, butyle, isobutyle ou terbutyle.

Le ligand peut être choisi :
- parmi les dérivés du phosphore, de l'arsenic ou de l'antimoine répondant en général aux formules :

   YRₘX₃₋ₘ, R₂Y - (CH₂)ₙ - YR₂, Y(OR)₃ et YOR₃,

   dans lesquelles Y = P, As ou Sb ; m = 0, 1, 2 ou 3 ; R = alkyle, aryle ou aryle substitué ; X = halogène, et n = 0, 1, 2, 3 ou 4 ; et
- parmi les ligands azotés tels que les amines et polyamines, l'imidazole, les imidazoles substitués, le pyrrole et les pyrroles substitués, les pyrazoles, les dérivés amidiques, les imines ou diimines (fabriqués par exemple par réaction du glyoxal avec un dérivé de l'aniline substitué sur le noyau aromatique), enfin les dérivés pyridiniques.

Des exemples particuliers de ligands sont ceux qui ont pour formules générales :

R-N=CR'-CR'=N-R, PR₃ et R₂P-(CH₂)ₙ-PR₂

avec R' = H ou CH₃, n = 1, 2, 3 ou 4 et R = alkyle, aryle ou aryle partiellement substitué par 1, 2, 3 ou 4 groupements méthyle, éthyle, isopropyle ou méthoxy.

Les formules développées suivantes illustrent certains de ces produits :

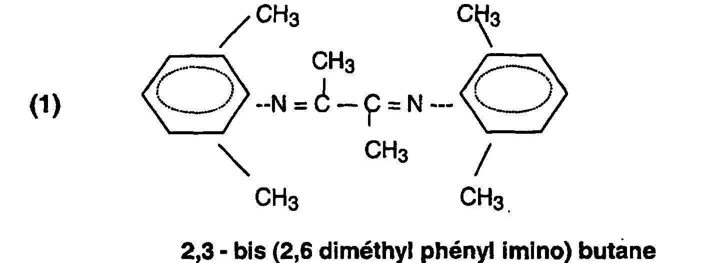

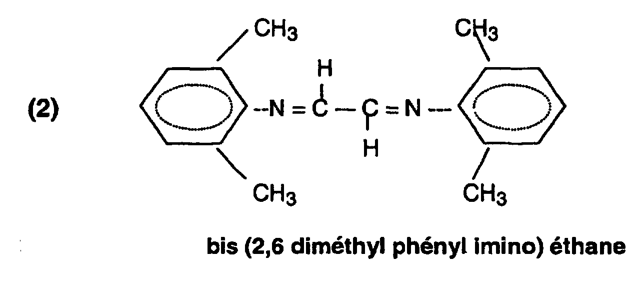

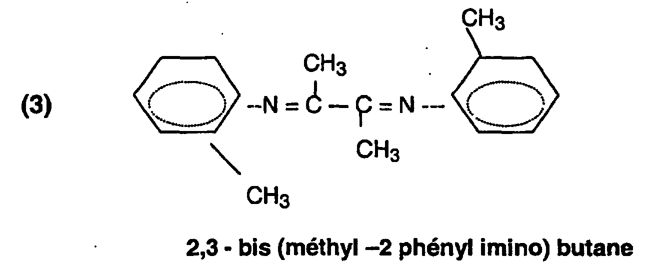

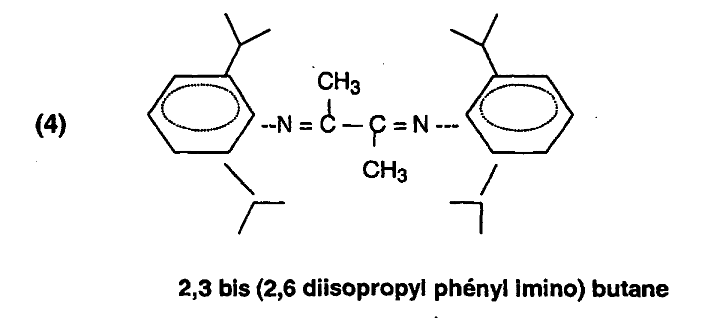

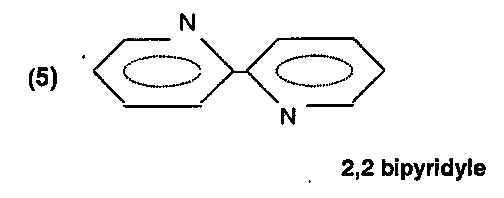

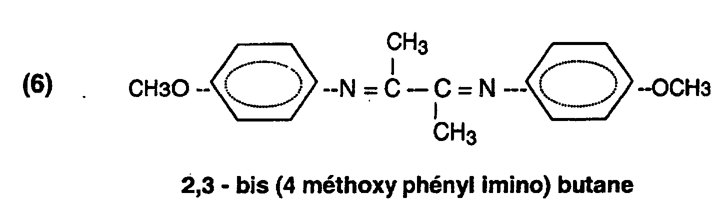

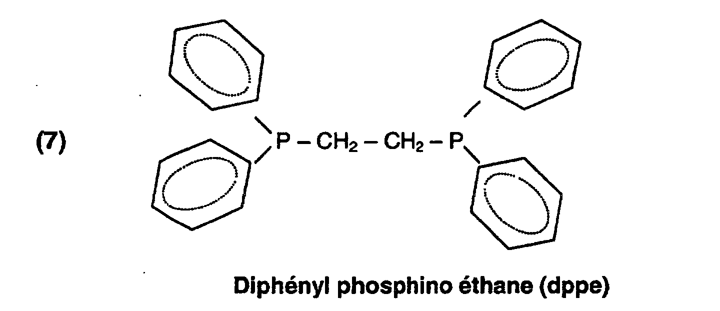

On peut éventuellement mettre en jeu un composé organique qui joue le rôle de solvant; comme solvants, on peut utiliser les hydrocarbures aliphatiques ou aromatiques, les éthers, les esters, les hydrocarbures halogénés et, à faible concentration, des sulfoxydes et des amides ; la réaction peut aussi être réalisée en l'absence de solvant ajouté ; c'est alors l'ester dont une partie ne réagit pas avec l'oléfine qui joue le rôle de solvant.

On peut encore adjoindre au catalyseur de codimérisation un sel d'un autre métal de transition (par exemple Co, Ni, Cu, Rh, Pd, Mn, Mo, W ou V, de préférence Ni, Cu, Rh ou Pd), introduit en proportion mineure par rapport au fer et qui permet d'accélérer la réaction, en particulier lorsque le substrat polyinsaturé à base de corps gras n'a pas ses doubles liaisons sous la forme conjuguée.

Le rapport molaire entre le ligand et le composé du fer est de préférence de 0,5 à 10, en particulier de 0,5 à 3.

Si le ligand est monocoordinant, on a intérêt à l'utiliser avec un rapport molaire ligand/métal de 2 à 3. Si le ligand est bicoordinant on l'utilisera plutôt avec un rapport molaire de 1 à 1,5. Le rapport molaire entre le réducteur et le composé du fer est généralement de 1 à 30, de préférence de 7 à 15.

Selon la présente invention, il est possible de préformer le système catalytique en faisant réagir le sel de fer, le ligand et le réducteur, puis de l'introduire dans le corps gras polyinsaturé en présence de l'oléfine.

Généralement, il est préférable d'ajouter le ligand au composé du fer en présence de corps gras insaturé et en présence d'oléfine, avant d'ajouter le réducteur. On peut aussi isoler un complexe réduit du fer FeHXL₂ (où L est une imine et X un halogène) ou FeHXL' (où L' est une diimine et X un halogène) et ajouter un réducteur tel qu'un alkylaluminium, ou autre, en présence du corps gras polyinsaturé.

La composition catalytique est ajoutée au système en quantité catalytique. Cette quantité s'exprime comme étant de 10⁻⁴ à 10⁻¹ mole de fer par mole de corps gras polyinsaturé conjugué. La température de réaction est de 40 à 120 °C et de préférence de 50 à 70 °C. La pression d'oléfine est de 0,1 à 30 MPa et de préférence de 2 à 5 MPa. Les temps de réaction dépendent de la concentration et de la nature du catalyseur. Les temps de réaction peuvent être courts, par exemple de quelques minutes à quelques heures.

On peut opérer selon un procédé continu ou discontinu. L'introduction du catalyseur et des esters dans le réacteur peut se réaliser en présence d'éthylène à basse température ou à plus haute température directement dans le réacteur.

Les corps gras ramifiés obtenus peuvent être hydrogénés pour obtenir des produits plus stables. L'hydrogénation des composés oléfiniques est effectuée au moyen d'un catalyseur connu pour hydrogéner les oléfines, soit par exemple, le nickel de Raney, le palladium sur charbon ou un nickel supporté, généralement après qu'on a éliminé le catalyseur de codimérisation par lavage à l'eau. On peut parfois utiliser le catalyseur de codimérisation comme catalyseur d'hydrogénation. Après hydrogénation, on élimine les composés saturés non ramifiés par cristallisation ou par distillation. On peut aussi distiller avant hydrogénation pour concentrer les produits ramifiés.

Les esters ramifiés peuvent servir de bases pour tensioactifs, émulsifiants, émollients, lubrifiants, ou subir d'autres traitements comme la transestérification avec des alcools plus lourds lorsqu'on a affaire, au départ, à des esters méthyliques. On peut encore les transformer en leurs sels de métaux lourds.

Les exemples suivants illustrent l'invention ; ils ne sont pas limitatifs.

### EXEMPLE 1

### Préparation du précurseur du catalyseur

Dans un tube de Schlenk, sous atmosphère d'argon, on introduit 0,1 mmole de tris-acétylacétonate de fer^{(III)} [Fe(acac)₃], 0,1 mmole de 2,3-bis (2,6-diméthylphénylimino) butane de formule R-N=CR'-CR'=N-R, avec R' = CH₃ et R = aryle partiellement substitué par 2 groupements méthyles, puis 20 ml d'ester méthylique conjugué de l'huile de tournesol dont la composition est la suivante : 7,1 % de C16:0, 4,0 % de C18:0, 29,9 % de C18:1, 2,9 % de C18:2 non conjugué et 56,1 % de C18:2 conjugué (33,8 mmoles). Cette suspension est chauffée à 60 °C pendant 30 minutes, ce qui conduit à une solution à laquelle on rajoute 1 mmole de triéthylaluminium (TEA), dilué à 10 % en volume dans le n-octane.

### Catalyse de codimérisation

Dans un autoclave de 250 ml en Hastelloy®, muni d'une agitation par barreau magnétique et d'une double enveloppe et préalablement chauffé à 60 °C, on introduit sous atmosphère d'argon, la totalité de la solution précédente. Le réacteur est alors mis sous une pression de 3 MPa d'éthylène maintenue constante au cours de la réaction. Au bout de 1 heure, l'agitation est arrêtée, le réacteur est dépressurisé et ouvert. Après élimination du catalyseur par lavage à l'eau acidulée, le mélange obtenu est analysé par chromatographie en phase vapeur sur colonne capillaire très polaire du type BPX 70 d'un diamètre de 0,32 cm et d'une longueur de 50 m. Les résultats figurent dans le tableau 1.

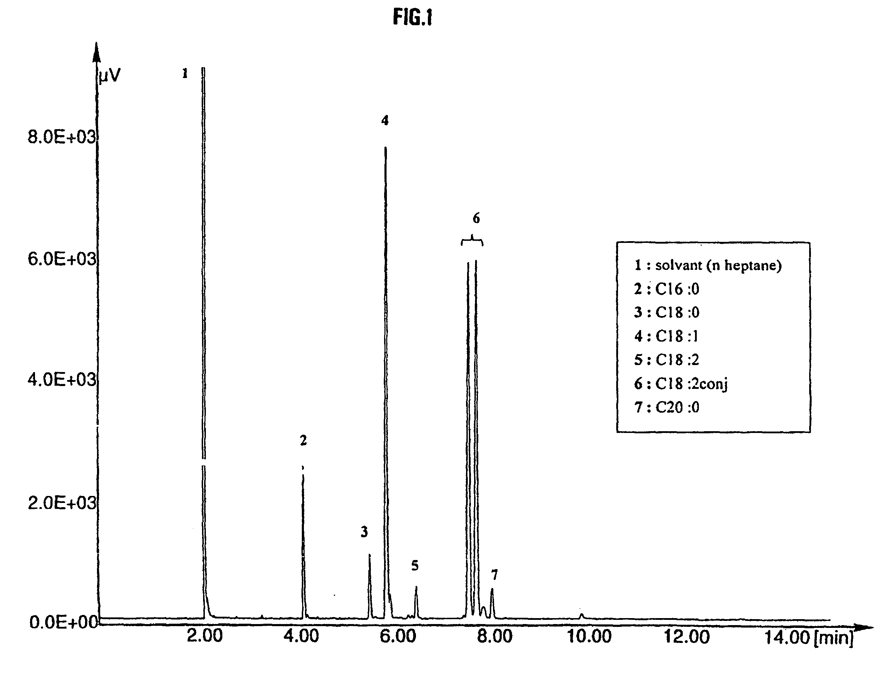

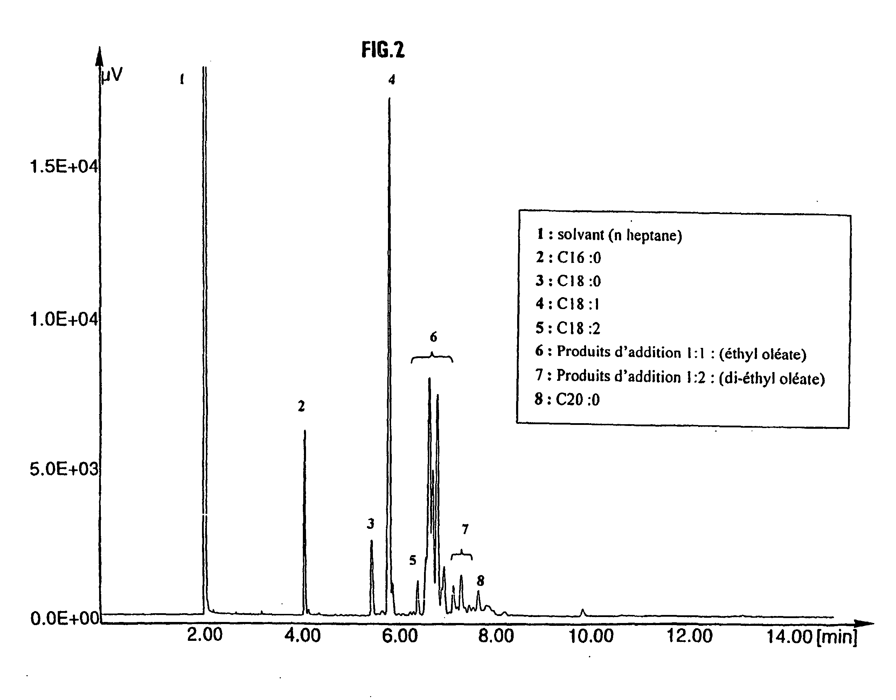

Le chromatogramme des produits de la réaction est donné en annexe (Fig. 2), ainsi que le chromatogramme du produit de départ (Fig. 1).

### EXEMPLE 2

Dans un autoclave de 250 ml en Hastelloy®, préalablement chauffé à 60 °C, on introduit la solution préparée dans l'Exemple 1, mais sans le TEA. Celui-ci (1 mmole dilué à 10 % en volume dans le n-octane) est introduit dans le réacteur à 60 °C sous atmosphère d'éthylène. Le réacteur est alors mis sous une pression constante de 3 MPa d'éthylène. La réaction est arrêtée au bout de 1 heure. Les résultats figurent dans le Tableau 1. Selon ce tableau, on n'observe pas de différence dans les deux manières d'opérer.

### EXEMPLE 3

On opère comme dans l'Exemple 1 mais on arrête la réaction après 2 heures d'agitation. Les résultats figurent dans le Tableau 1.

### EXEMPLE 4 (comparatif)

On opère comme dans l'Exemple 1 mais sans utiliser de ligand. La réaction est arrêtée au bout de 1 heure. Les résultats figurent dans le Tableau 1.

### EXEMPLE 5

On opère comme dans l'Exemple 1, mais la réaction catalytique est réalisée à 90 °C. La réaction est arrêtée au bout de 1 heure. Les résultats figurent dans le Tableau 1.

### EXEMPLE 6

On opère comme dans l'Exemple 5, mais sous une pression d'éthylène de 1 MPa. La réaction est arrêtée au bout de 1 heure. Les résultats figurent dans le Tableau 1.

### EXEMPLE 7

On opère comme dans l'Exemple 6, mais on utilise un rapport molaire réducteur/métal de 7 à la place de 10. La réaction est arrêtée au bout de 1 heure. Les résultats figurent dans le Tableau 1.

### EXEMPLE 8

On opère comme dans l'Exemple 5 mais sous une pression d'éthylène de 0,1 MPa et en utilisant 4 fois plus (en mmoles) de tous les réactifs rentrant dans la composition du catalyseur, soit 0,4 mmole de tris-acétylacétonate de fer^{(III)} [Fe(acac)₃], 0,4 mmole de 2,3-bis (2,6-diméthylphényl imino) butane et 4 mmoles de triéthylaluminium (TEA), dilué à 10 % en volume dans le n-octane. La réaction est arrêtée au bout de 1 heure. Les résultats figurent dans le Tableau 1.

### EXEMPLE 9

On opère comme dans l'Exemple 5, mais du triisobutylaluminium est utilisé comme agent réducteur, à la place du TEA, à raison de 1,2 mmoles / 0,1 mmole de fer. L'ester méthylique de tournesol est de même composition que celui utilisé et décrit dans l'Exemple 1. La réaction est arrêtée au bout de 1 heure. Les résultats figurent dans le Tableau 1.

### EXEMPLE 10

On opère comme dans l'Exemple 5, mais le réducteur employé est de l'hydrure de diisobutylaluminium, à raison de 2,2 mmoles / 0,1 mmole de fer. La réaction est arrêtée au bout de 1 heure. Les résultats figurent dans le Tableau 1.

### EXEMPLE 11

On opère comme dans l'Exemple 5 mais à une température de 85 °C, en utilisant comme agent réducteur du butyl-octyl-magnésium à raison de 60 mmoles / 0,1 mmole de fer. La réaction est arrêtée au bout de 2 heures. Les résultats figurent dans le Tableau 1.

### EXEMPLE 12

On opère comme dans l'Exemple 1 mais on utilise de l'octoate de fer^{(III)} [Fe(octoate)₃] à la place du Fe(acac)₃. La réaction est arrêtée au bout de 1 heure. Les résultats figurent dans le Tableau 1.

### EXEMPLE 13

On opère comme dans l'Exemple 1 mais on utilise comme ligand le diphénylphosphino éthane (dppe) à la place du 2,3-bis (2,6-diméthyl phényl imino) butane. La réaction est arrêtée au bout de 1 heure. Les résultats figurent dans le Tableau 1.

### EXEMPLE 14

On opère comme dans l'Exemple 1 mais on utilise comme ligand le 2,3-bis (2-méthyl phényl imino) butane à la place du 2,3-bis (2,6-diméthyl phényl imino) butane. La réaction est arrêtée au bout de 1 heure. Les résultats figurent dans le Tableau 1.

### EXEMPLE 15

On opère comme dans l'Exemple 1 mais on utilise comme ligand le bis (2,6-diméthyl phényl imino) éthane à la place du 2,3-bis (2,6-diméthyl phényl imino) butane. La réaction est arrêtée au bout de 1 heure. Les résultats figurent dans le Tableau 1.

### EXEMPLE 16

On opère comme dans l'Exemple 1 mais on utilise comme ligand 2,3-bis (4-méthoxy phényl imino) butane à la place du 2,3-bis (2,6-diméthyl phényl imino) butane. La réaction est arrêtée au bout de 1 heure. Les résultats figurent dans le Tableau 1.

### EXEMPLE 17

On opère comme dans l'Exemple 1 mais on utilise comme ligand le 2,3-bis(2,6-diisopropyl phényl imino) butane à la place du 2,3-bis (2,6-diméthyl phényl imino) butane. La réaction est arrêtée au bout de 1 heure. Les résultats figurent dans le Tableau 1.

### EXEMPLE 18

On opère comme dans l'Exemple 1 mais on utilise comme ligand la 2,2-bipyridyl à la place du 2,3-bis (2,6-diméthyl phényl imino) butane. La réaction est arrêtée au bout de 1 heure. Les résultats figurent dans le Tableau 1.

### EXEMPLE 19

On opère comme dans l'Exemple 1, mais on utilise une quantité 10 fois plus grande de tous les réactifs mis en jeu ; soit 200 ml d'ester méthylique de tournesol conjugué, 1 mmole de Fe(acac)₃, 1 mmole de ligand 2,3-bis (2,6-diméthyl phényl imino) butane et 10 mmoles d'agent réducteur (AlEt₃) dilué à 10% en volume dans du n-octane. La réaction est arrêtée au bout de 3 heures. Les résultats figurent dans le Tableau 1.

L'analyse chromatographique en phase vapeur met en évidence une très grande sélectivité en produit de mono-addition. On n'observe généralement pas plus de 3,5 % de produit de di-addition.

### EXEMPLE 20 (comparatif)

On opère comme dans l'Exemple 1 mais en utilisant un système catalytique constitué de 0,1 mmole de bis-acétylacétonate de cobalt et 0,1 mmole de diphénylphosphinoéthane et 3 mmoles de diéthylchloroaluminium. La réaction est arrêtée au bout de 1 heure. Les résultats figurent dans le Tableau 1.

### EXEMPLE 21 (comparatif)

On opère comme dans l'Exemple 1 mais en introduisant sous atmosphère d'éthylène 2 mmoles de triéthylaluminium (TEA) dilué à 10% en volume dans le n-octane, au lieu de 1 mmole, avec addition ultérieure de 0,1 mmole d'octoate de nickel dilué dans 5 ml de n-octane. La réaction est arrêtée au bout de 4 heures. Les résultats figurent dans le Tableau 1.

### EXEMPLE 22

Le produit de réaction issu de l'Exemple 19, après avoir subi un traitement approprié pour éliminer la totalité des sels d'aluminium ainsi que le ligand azoté, est hydrogéné totalement en utilisant 100 mg d'un catalyseur palladium sur charbon.

Le mélange d'esters méthyliques saturés obtenu après filtration du palladium sur charbon est purifié par 2 opérations successives de cristallisation dans de l'acétone à -20 °C. Après élimination de la majorité du palmitate et du stéarate de méthyle, le composé d'addition après évaporation de l'acétone reste liquide jusqu'à une température de -30 °C.

La chromatographie en phase vapeur indique que le composé contient 3,3 % de palmitate de méthyle, 1,0 % de stéarate de méthyle, 91,5 % de produits de monoaddition, 3,2 % de produits de di-addition et 1 % de béhénate de méthyle.

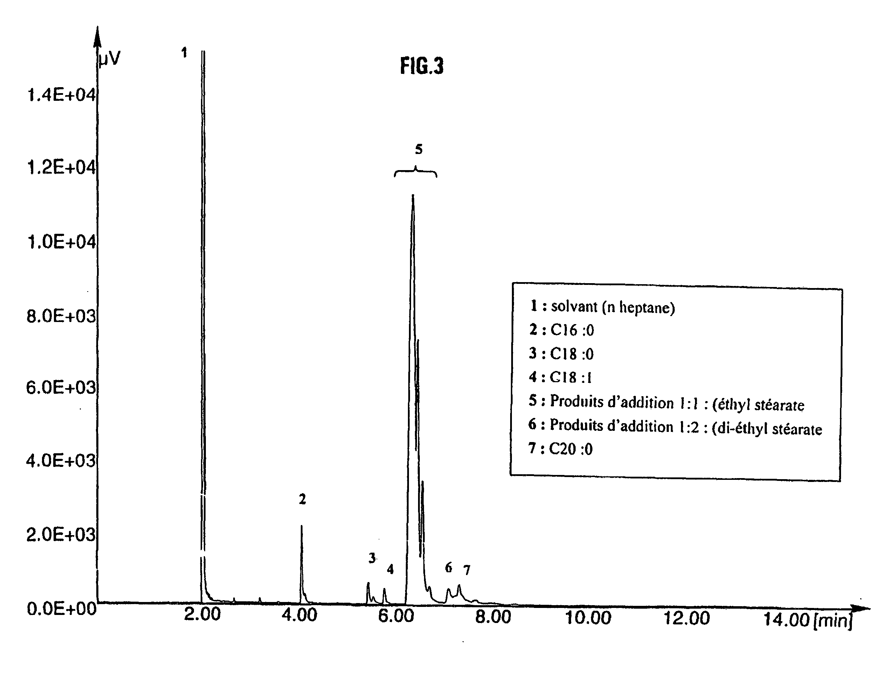

Le chromatogramme du produit purifié est donné en annexe (Fig. 3)

La RMN de l'hydrogène donne un signal de proton à 1,8 ppm correspondant à 1 proton sur un carbone tertiaire. C'est principalement la RMN du produit avant hydrogénation qui donne plusieurs indications sur le groupement vinylique entre 4,9 et 5 ppm, absent dans le produit de départ et correspondant à une double liaison terminale, soit un composé de type vinyloctadécénoate de méthyle.

Le Tableau 1 regroupe les résultats des Exemples 1 à 21, qui concernent l'addition de l'éthylène sur un ester méthylique d'huile de tournesol conjugué. Dans ce Tableau 1, on a considéré la conversion des linoléates conjugués et la conversion en produit d'addition par rapport à l'ester conjugué initial. Le catalyseur est un sel de fer réduit généralement par AlEt₃ dilué dans le n-octane, sauf lorsqu'il est indiqué un autre solvant ou un autre réducteur.

**TABLEAU 1**

| Addition de l'éthylène sur un ester méthylique d'huile de tournesol conjugué | | |
|---|---|---|
| Exemple | Conversion des C18 :2 conjugués (% en poids) | Conversion en produits de mono-addition (% en poids) |
| 1 | 83 | 80 |
| 2 | 82 | 80 |
| 3 | 100 | 97 |
| 4 | 0 | 0 |
| 5 | 60 | 59 |
| 6 | 49 | 49 |
| 7 | 27 | 27 |
| 8 | 72 | 71 |
| 9 | 40 | 40 |
| 10 | 22 | 22 |
| 11 | 10 | 10 |
| 12 | 80 | 79 |
| 13 | 22 | 22 |
| 14 | 26 | 26 |
| 15 | 5 | 5 |
| 16 | 21 | 21 |
| 17 | 8 | 8 |
| 18 | 12 | 12 |
| 19 | 98 | 95 |
| 20 | 4 | 4 |
| 21 | 100 | 68* |

| | | |
|---|---|---|
| * *le complément (32%) étant les produits de di-addition.* | | |

### Commentaires sur les résultats obtenus

### Influence de la température

Les résultats des Exemples de 1 à 3 qui ont fonctionné à une température de 60 °C donnent des conversions plus élevées que celle obtenue dans l'Exemple 5, qui a travaillé à une température de 90 °C.

### Influence de la pression

L'Exemple 6 fonctionnant à une pression de 1 MPa est moins performant que les Exemples 1 à 3 qui ont travaillé sous 3 MPa de pression d'éthylène.

### Influence du ligand

L'Exemple 4 (comparatif) montre qu'un système catalytique sans ligand est totalement inactif.

L'Exemple 13 utilisant un ligand phosphoré du type diphényl-phosphinoéthane est nettement moins performant que le 2,3-bis (2,6-diméthyl phényl imino) butane utilisé dans l'Exemple 1.

Les systèmes catalytiques des Exemples de 14 à 18 utilisant des ligands azotés sont tous moins performants que les Exemples 1 à 3 utilisant le 2,3-bis (2,6-diméthyl phényl imino) butane.

### Influence du réducteur

Une stoechiométrie en aluminium sur métal voisine de 10 (Exemples de 1 à 3) donne de meilleurs résultats qu' une stoechiométrie inférieure, voisine de 7 (Exemple 7).

Les Exemples 9, 10 et 11 (comparatifs avec l'Exemple 1), qui ont utilisé d'autres réducteurs que le triéthylaluminium, demeurent moins performants.

### Nature du sel de fer

L'essai 12 qui a utilisé de l'octoate de fer (III) en lieu et place de l'acétylacétonate de fer (III) donne des résultats identiques.

### Activité catalytique

Comparaison de l'activité du système catalytique à base de fer de l'invention (Exemple 1) avec celui au cobalt (Exemple 20) montre une activité 20 fois plus grande pour le système à base de fer à iso-concentration en métal.

## Revendications

1. Procédé d'obtention d'un codimère **caractérisé en ce que** l'on additionne au moins un composé monooléfinique sur un corps gras comportant au moins deux liaisons éthyléniques conjuguées ou non conjuguées en présence d'un système catalytique comprenant au moins un composé réducteur, au moins un ligand contenant du phosphore, de l'arsenic, de l'antimoine ou de l'azote, au moins un composé du fer et éventuellement au moins un sel d'un autre métal de transition en proportion mineure par rapport au fer.

2. Procédé selon la revendication 1 **caractérisé en ce que** dans ledit système catalytique :
- ledit composé du fer répond à la formule Fe Xₙ, dans laquelle n = 2 ou 3, et X représente un halogénure, un thiocyanure, un sulfate, un nitrate, un alcoolate, un carbonate, un carboxylate, une dicétone, un ester d'acide bétacétocarboxylique, un hydroxyle, un groupe alkyle ou alkényle ou un hydrure ;
- ledit réducteur répond à l'une des formules AlRₓ(X)₃₋ₓ, LiAlH₄, NaBH₄ ou LiAlHₙ(OR)₄₋ₙ dans lesquelles R = alkyle, X = halogénure et n = 1, 2 ou 3, ou consiste en un aluminoxane ou un composé organo-magnésien ;
- et ledit ligand est choisi parmi les dérivés du phosphore, de l'arsenic ou de l'antimoine répondant en général aux formules :
YRₘX₃₋ₘ, R₂Y - (CH₂)ₙ - YR₂, Y(OR)₃ et YOR₃,
dans lesquelles Y = P, As ou Sb ; m = 0, 1, 2 ou 3 R = alkyle, aryle ou aryle substitué ; X = halogène, et n = 0, 1, 2, 3 ou 4 ;
et parmi les ligands azotés, amines et polyamines, imidazole, imidazoles substitués, pyrrole et pyrroles substitués, pyrazoles, dérivés amidiques, imines diimines et dérivés pyridiniques.

3. Procédé selon la revendication 1 ou 2 **caractérisé en ce que** ledit composé monooléfinique est choisi parmi les hydrocarbures monooléfiniques comme l'éthylène, le propylène ou le butène-1.

4. Procédé selon l'une des revendications 1 à 3 **caractérisé en ce que** ledit corps gras comportant au moins deux liaisons éthyléniques est choisi parmi les corps gras diéniques ou polyéniques conjugués ou conjugables, le nombre d'atomes de carbone de la chaîne grasse comportant de 18 à 26 atomes de carbone sur la chaîne qui porte le groupe carboxylique, celui-ci étant lié à un alcool mono, di, tri ou tétrafonctionnel de 1 à 18 atomes de carbone.

5. Procédé selon l'une des revendications de 1 à 4 **caractérisé en ce que** le système catalytique comporte, à titre de composé du fer, des halogénures, des acétylacétonates ou des carboxylates et, à titre de réducteur, un système à base d'alkylaluminium, substitué ou non, ou de l'aluminoxane ou des hydrures d'aluminium ou de bore.

6. Procédé selon l'une des revendications de 1 à 5 **caractérisé en ce que** le ligand introduit est une diimine ayant pour formule R-N=CR'-CR'=N-R, avec R' = H ou CH3 et R = alkyle, aryle ou aryle partiellement substitué par 1, 2 ou 3 groupements méthyle, éthyle, isopropyle ou méthoxy.

7. Procédé selon la revendication 6 **caractérisé en ce que** le ligand est le 2,3-bis(2,6-dimethyl phényl imino) butane.

8. Procédé selon l'une des revendications 1 à 7 **caractérisé par le fait que** le catalyseur est un système mixte obtenu par réaction d'une part d'un complexe FeHXL₂ ou FeHXL', où X est un anion, halogène ou carboxylate, L est une imine et L' est une diimine, introduit dans un rapport molaire 1:2 par rapport au fer pour le complexe au fer contenant L, et dans un rapport molaire 1:1 par rapport au fer pour le complexe au fer contenant L' et d'autre part un réducteur.

9. Procédé selon l'une des revendications de 1 à 8 **caractérisé en ce que** la concentration en fer est de 10⁻⁴ à10⁻¹ mole de fer par mole de corps gras polyinsaturé conjugué, la température de réaction est de 40 à 120 °C, la pression en oléfine est de 0,1 à 30 MPa, le rapport molaire entre le ligand et le fer est de 0,5 à 10 et le rapport molaire entre le réducteur et le fer est de 1 à 30.

10. Procédé selon l'une des revendications de 1 à 9 **caractérisé en ce que** l'ester engagé est préalablement conjugué avec un système de type alcoolate alcalin ou conjugué au cours de l'addition avec le même système que celui qui permet l'addition d'oléfine.

11. Procédé selon la revendication 10 **caractérisé en ce que** l'ester engagé est conjugué au cours de l'addition d'oléfine ou en la précédant, avec un catalyseur au fer co-catalysé par des traces d'au moins un métal de transition choisi parmi le nickel, le cuivre, le rhodium et le palladium.

12. Procédé selon l'une des revendications 1 à 11 **caractérisé par le fait que** le composé oléfinique produit par codimérisation contient un chaînon ou plusieurs chaînons dont la longueur correspond à l'oléfine engagée.

13. Procédé de préparation de composés ramifiés saturés par hydrogénation de composés ramifiés obtenus par un procédé selon l'une des revendications 1 à 12, **caractérisé par le fait que** les composés ramifiés insaturés sont hydrogénés en présence du catalyseur de codimérisation ou d'un catalyseur connu d'hydrogénation, éventuellement après filtration ou élimination du catalyseur de codimérisation.

14. Procédé selon la revendication 13 **caractérisé par le fait que** le composé ramifié saturé obtenu par hydrogénation est ensuite purifié après séparation des composés ramifiés par distillation et/ou par élimination des composés saturés non ramifiés par cristallisation dans un solvant.

## Claims

1. Process for obtaining a codimer, **characterized in that** at least one monoolefinic compound is added to a fatty substance that comprises at least two conjugated or unconjugated ethylene bonds, in the presence of a catalytic system that comprises at least one reducing compound, and at least one ligand that contains phosphorus, arsenic, antimony or nitrogen, at least one iron compound and optionally at least one salt of another transition metal, in a minor proportion with respect to iron.

2. Process according to claim 1, **characterized in that** in said catalytic system:
-- said iron compound corresponds to formula Fe Xₙ, in which n = 2 or 3, and X represents a halide, a thiocyanide, a sulfate, a nitrate, an alcoholate, a carbonate, a carboxylate, a diketone, a betacetocarboxylic acid ester, a hydroxyl, an alkyl or alkenyl group or a hydride;
-- said reducing agent corresponds to one of formulas AlRₓ(X)₃₋ₓ, LiAlH₄, NaBH₄ or LiAlHₙ(OR)₄₋ₙ, in which R = alkyl, X = halide and n = 1, 2 or 3, or consists of an aluminoxane or an organo-magnesium compound;
-- and said ligand is selected from among the derivatives of phosphorus, arsenic or antimony that correspond in general to the formulas:
YRₘX₃₋ₘ, R₂Y-(CH₂)ₙ-YR₂, Y(OR)₃ and YOR₃,
in which Y = P, As or Sb; m = 0, 1, 2, or 3; R = alkyl, aryl or substituted aryl; X = halogen, and n = 0, 1, 2, 3 or 4;
and from among the nitrogen-containing ligands, amines and polyamines, imidazole, substituted imidazoles, pyrrole and substituted pyrroles, pyrazoles, amidic derivatives, imines, diimines, and pyridinic derivatives.

3. Process according to claim 1 or 2, **characterized in that** said monoolefinic compound is selected from among the monoolefinic hydrocarbons such as ethylene, propylene, or butene-1.

4. Process according to one of claims 1 to 3, **characterized in that** said fatty substance that comprises at least two ethylene bonds is selected from among the diene or polyene fatty substances that are conjugated or can be conjugated, whereby the number of carbon atoms of the fatty chain comprises 18 to 26 carbon atoms on the chain that carries the carboxylic group, with the latter being linked to a mono-, di-, tri- or tetrafunctional alcohol with 1 to 18 carbon atoms.

5. Process according to one of claims 1 to 4, **characterized in that** the catalytic system comprises, as an iron compound, halides, acetylacetonates, or carboxylates and, as a reducing agent, a system with an alkylaluminum base, which may or may not be substituted, or aluminoxane or aluminum or boron hydrides.

6. Process according to one of claims 1 to 5, **characterized in that** the ligand that is introduced is a diimine that has as its formula R-N=CR'-CR'=N-R, with R' = H or CH3 and R = alkyl, aryl or aryle that is partially substituted by 1, 2 or 3 methyl, ethyl, isopropyl or methoxy groups.

7. Process according claim 6, **characterized in that** the ligand is 2,3-bis(2,6 dimethylphenylimino)butane.

8. Process according to one of claims 1 to 6, **characterized in that** the catalyst is a mixed system that is obtained by reaction, on the one hand, of an FeHXL₂ or FeHXL' complex, where X is an anion, halogen, or carboxylate, L is an imine and L' is a diimine, introduced in a 1:2 molar ratio relative to the iron for the iron complex that contains L, and in a 1:1 molar ratio relative to the iron for the iron complex that contains L' and, on the other hand, a reducing agent.

9. Process according to one of claims 1 to 7, **characterized in that** the iron concentration is 10⁻⁴ to 10⁻¹ mol of iron per mol of conjugated polyunsaturated fatty substance, the reaction temperature is 40 to 120°C, and the olefin pressure is 0.1 to 30 MPa, the molar ratio between the ligand and the iron is 0.5 to 10 and the molar ratio between the reducing agent and the iron is 1 to 30.

10. Process according to one of claims 1 to 9, **characterized in that** the ester that is employed is conjugated in advance with an alkaline alcoholate-type system or conjugated during the addition with the same system as the one that makes possible the addition of olefin.

11. Process according to claim 10, **characterized in that** the ester that is employed is conjugated during the addition of olefin or before with an iron catalyst that is co-catalyzed by traces of at least one transition metal that is selected from among nickel, copper, rhodium and palladium.

12. Process according to one of claims 1 to 11, **characterized in that** the olefinic compound that is produced by codimerization contains one or more links whose length corresponds to the olefin that is employed.

13. Process for preparation of saturated branched compounds by hydrogenation of branched compounds obtained by a process according to one of claims 1 to 12, **characterized in that** the unsaturated branched compounds are hydrogenated in the presence of the codimerization catalyst or a known hydrogenation catalyst, optionally after filtration or elimination of the codimerization catalyst.

14. Process according to claim 13, **characterized in that** the saturated branched compound that is obtained by hydrogenation is then purified after the branched compounds are separated by distillation and/or by elimination of the unbranched saturated compounds by crystallization in a solvent.

## Patentansprüche

1. Verfahren zum Erhalt eines Codimers, **dadurch gekennzeichnet, dass** man wenigstens eine monoolefinische Verbindung auf einen Fettkörper, der wenigstens zwei konjugierte oder nicht konjugierte ethylenische Bindungen enthält, in Gegenwart eines katalytischen Systems zugibt, das wenigstens eine reduzierende Verbindung, wenigstens einen Ligand, der Phosphor, Arsen, Antimon oder Stickstoff enthält, wenigstens eine Eisenverbindung und gegebenenfalls wenigstens ein Salz eines anderen Übergangsmetalls in einem untergeordneten Anteil im Verhältnis zum Eisen umfasst.

2. Verfahren nach Anspruch 1, **dadurch gekennzeichnet, dass** in dem katalytischen System
- die Eisenverbindung, die der Formel FeXₙ entspricht, in der n= 2 oder 3 und X ein Halogenid, ein Thiocyanid, ein Sulfat, ein Nitrat, ein Alkoholat, ein Carbonat, ein Carboxylat, ein Diketon, ein beta-Ketocarboxylsäureester, ein Hydroxl, eine Alkyl- oder Alkenylgruppe oder ein Hydrid darstellt;
- das Reduktionsmittel einer der Formeln AlRₓ(X)₃₋ₓ, LiAlH₄, NaBH₄ oder LiAlHₙ(OR)₄₋ₙ, in denen R = Alkyl, X = Halogenid und n = 1, 2 oder 3 ist, entspricht oder aus einem Aluminoxan oder einer organischen Magnesiumverbindung besteht;
- und der Ligand gewählt wird unter den Derivaten des Phosphors, des Arsens oder des Antimons, die im allgemeinen den Formeln entsprechen:
YRₘX₃₋ₘ, R₂Y-(CH₂)ₙ-YR₂, Y(OR)₃ und YOR₃,
in denen Y = P, As oder Sb; m = 0, 1, 2 oder 3; R = Alkyl, Aryl oder substituiertes Aryl, X = Halogen und n = 0, 1, 2, 3 oder 4 ist;
und unter den stickstoffhaltigen Liganden, Aminen und Polyaminen, Imidazol, substituierten Imidazolen, Pyrrol und substituierten Pyrrolen, Pyrazolen, Amidderivatien, Iminen, Diiminen und Pyridinderivaten.

3. Verfahren nach Anspruch 1 oder 2, **dadurch gekennzeichnet, dass** die monoolefinische Verbindung gewählt wird unter den monoolefinischen Kohlenwasserstoffen wie Ethylen, Propylen oder 1-Buten.

4. Verfahren nach einem der Ansprüche 1 bis 3, **dadurch gekennzeichnet, dass** der Fettkörper, der wenigstens zwei ethylenische Bindungen umfasst gewählt wird unter den konjugierten oder konjugierbaren dienischen oder polyenischen Fettkörpern, wobei die Anzahl von Kohlenstoffatomen der Fettkette 18 bis 26 Kohlenstoffatome auf der Kette umfasst, die die Carboxylgruppe trägt, wobei diese an einen mono-, di-, tri- oder tretrafunktionellen Alkohol mit 1 bis 18 Kohlenstoffatomen gebunden ist.

5. Verfahren nach einem der Ansprüche 1 bis 4, **dadurch gekennzeichnet, dass** das katalytische System als Eisenverbindung Halogenide, Acetylacetonate oder Carboxylate und als Reduktionsmittel ein System auf Basis von Alkylaluminium, ggf. substituiert, oder von Aluminoxan oder Aluminiumoder Borhydriden umfasst.

6. Verfahren nach einem der Ansprüche 1 bis 5, **dadurch gekennzeichnet, dass** der eingeführte Ligand ein Diimin ist, das als Formel R-N=CR'-CR'=N-R mit R' = H oder CH₃ und R = Alkyl, Aryl oder Aryl, das teilweise durch 1, 2 oder 3 Methyl-, Ethyl-, Isopropyl- oder Methoxygruppen substituiert ist, hat.

7. Verfahren nach Anspruch 6, **dadurch gekennzeichnet, dass** der Ligand 2,3-bis-(2,6-Dimehtylphenylimino)-Butan ist.

8. Verfahren nach einem der Ansprüche 1 bis 7, **gekennzeichnet durch** die Tatsache, dass der Katalysator ein gemischtes System ist, das **durch** Reaktion von einerseits einem FeHXL₂- oder FeHXL'-Komplex erhalten wird, worin X ein Halogen- oder Carboxylatanion ist, L ein Imin und L' ein Diimin ist, was in einem 1:2-Molverhältnis im Verhältnis zum Eisen für den L enthaltenden Eisenkomplex und in einem 1:1-Molverhältnis im Verhältnis zum Eisen für den L' enthaltenden Eisenkomplex eingeführt ist, und andererseits einem Reduktionsmittel erhalten wird.

9. Verfahren nach einem der Ansprüche 1 bis 8, **dadurch gekennzeichnet, dass** die Eisenkonzentration 10⁻⁴ bis 10⁻¹ mol Eisen pro mol ungesättigten, konjugierten Fettkörper ist, die Reaktionstemperatur 40 bis 120°C ist, der Olefindruck 0,1 bis 30 MPa ist, das Molverhältnis zwischen dem Ligand und Eisen 0,5 bis 10 ist und das Molverhältnis zwischen dem Reduktionsmittel und dem Eisen 1 bis 30 ist.

10. Verfahren nach einem der Ansprüche 1 bis 9, **dadurch gekennzeichnet, dass** der eingesetzte Ester vorher mit einem System vom Typ alkalisches Alkoholat konjugiert wird oder während der Zugabe mit demselben System konjugiert wird, das die Olefinzugabe ermöglicht.

11. Verfahren nach Anspruch 10, **dadurch gekennzeichnet, dass** der eingesetzte Ester während der Olefinzugabe oder ihr vorangestellt mit einem Katalysator mit Eisen, cokatalysiert durch Spuren von wenigstens einem Übergangsmetall konjugiert wird, das unter Nickel, Kupfer, Rhodium und Palladium gewählt ist.

12. Verfahren nach einem der Ansprüche 1 bis 11, **gekennzeichnet durch** die Tatsache, dass die **durch** Codimerisierung erzeugte olefinische Verbindung eine oder mehrere Kettenabschnitte enthält, deren Länge dem eingesetzten Olefin entspricht.

13. Verfahren zur Herstellung verzweigter Verbindungen, die durch Hydrierung von verzweigten Verbindungen gesättigt sind, die durch ein Verfahren gemäß einem der Ansprüche 1 bis 12 erhalten sind, **gekennzeichnet durch** die Tatsache, dass die ungesättigten verzweigten Verbindungen in Gegenwart eines Codimerisierungskatalysators oder eins bekannten Hydrierungskatalysators ggf. nach Filtrierung oder Entfernung des Codimerisierungskatalysators hydriert werden.

14. Verfahren nach Anspruch 13, **gekennzeichnet durch** die Tatsache, dass die ungesättigte, verzweigte, **durch** Hydrierung erhaltene Verbindung anschließend nach Trennung der verzweigten Verbindungen **durch** Destillation und/oder **durch** Entfernung der nicht verzweigten, gesättigten Verbindungen **durch** Kristallisation in einem Lösungsmittel gereinigt wird.
